# EUROPEAN PATENT SPECIFICATION

(11) **EP 1 144 980 B1**
(45) Date of publication and mention of the grant of the patent: **22.08.2007**
(21) Application number: 99962470.3
(22) Date of filing: 24.12.1999
(51) Int. Cl.: G01N 15/02, G01N 33/80, G01N 33/49, G01N 33/86

(54) **A METHOD OF TESTING A CELL SAMPLE**
EIN VERFAHREN ZUR UNTERSUCHUNG EINER ZELLPROBE
PROCEDE D'ANALYSE D'UN ECHANTILLON DE GLOBULES

(30) Priority: 29.12.1998 GB 9828765
(43) Date of publication of application: 17.10.2001
(73) Proprietor: Shine, Ian, Basil, New York, NY 10025 (US); Shine, Thomas, Adam, New Haven, CT 06511 (US)
(72) Inventor: Shine, Ian, Basil, New York, NY 10025 (US); Shine, Thomas, Adam, New Haven, CT 06511 (US)
(74) Representative: Finnie, Peter John
(86) International application number: PCT/GB1999/004438
(87) International publication number: WO 2000/039560

(56) References cited:
- WO-A-97/24601
- GB-A- 2 186 684
- US-A- 4 436 827
- US-A- 4 900 685
- US-A- 5 064 765
- US-A- 5 350 652
- US-A- 5 510 261

## Description

### Background to the Invention

All types of blood cells occasionally agglutinate spontaneously, frequently heralding a serious haemolytic disease. It may indicate an underlying malignancy such as non-Hodgkin's lymphoma, Hodgkin's disease, acute lymphocytic leukaemia, carcinoma, thymoma and ovarian tumours. It occurs in blood group incompatibility as in haemolytic disease of the newborn, and mis-matched blood transfusions; also in paraoxysmal nocturnal haemoglobinuria and hypogammaglobulinemia; in some collagen diseases such as disseminated lupus erythematosis, rheumatoid arthritis, ulcerative colitis and hepatitis; in some infections such as viral and Mycoplasma pneumonia, cytomegalovirus, tuberculosis and infectious mononucleosis, and as a toxic reaction to some drugs such as L-dopa. As the presence of intra or extra vascular haemolysis in these diseases carries at least a 10% mortality, the identification of red cell agglutination is useful for the early diagnosis and for monitoring the response to treatment.

Traditionally, agglutination is detected by visually observing clumped cells [e.g. US-A-4 436 827. Whilst automated cell counters have supplanted all manual routine haematology they cannot detect agglutination sufficiently accurately to avoid manual verification. Indeed, existing automated cell counters erroneously measure agglutinated clumps of cells as one large cell producing an inaccurate mean cell volume and cell count and compound indices derived from them. An abnormally high mean corpuscular volume (MCV) or an abnormally elevated mean corpuscular haemoglobin concentration (MCHC) displayed by commercial haematology autoanalysers alerts the technician to the possibility of the presence of agglutination. However, these indices are inadequate indicators of agglutination because they are not specific, moreover agglutination must rise to high levels before the indices exceed the normal limits. An elevated MCHC is produced by red cell fragmentation, lymphocytosis, hyperglycemia and haemoglobinaemia and therefore requires manual inspection and further testing to establish the diagnosis.

In conventional laboratories which perform blood typing and cross-matching, to determine the blood group of a sample one or two drops of existing commercially available blood group antibodies are added to neat whole blood, or more usually a 3 to 5 % suspension of whole blood in normal saline. The suspension is incubated at room temperature for some minutes, typically 2 or 3 minutes. The suspension is then centrifuged for 30 to 45 seconds at 3000 rpm in a bench-top centrifuge. The suspension is then gently shaken for a few seconds. The tube is then examined visually for the presence of agglutinated cells and confirmed using low-powered microscopy. Cross-matching is performed in the same way using the recipient's plasma as the antibody in place of commercial antibody to confirm that there is no agglutination.

### Summary of the Invention

According to the present invention, a method of detecting agglutination in a sample of cells comprises the steps of inducing cells to change at least one of their properties so as to separate agglutinated cells and detecting the resultant alteration in the cell population.

Preferably, the property change is that of the shape of the cells. More preferably, the cell sample is subject to an alteration in environment to cause the cells to sphere. In a preferred example, the alteration in the environment is a change in osmolality of a liquid medium in which the cells are suspended, preferably by the addition of water.

Preferably, alterations in the cell population are detected by passing one or more aliquots of the cell sample through a sensor which is adapted to count the number of cells passing through the sensor. More preferably, the sample is fed continuously into a solution the osmolality of which is changed continuously to produce a continuous series of aliquots of cells which are passed through the sensor.

Preferably, the method further comprises the step of pre-treating the sample of cells to induce, or at least attempt to induce, agglutination. In one preferred use of the invention, a cell sample of unknown antigenicity from one source is mixed with antibodies from a different source. The antibodies may be manufactured or come from whole blood, plasma or typically serum. In a further step, the putative antigen-antibody mixture may be tested at different temperatures to reveal heat sensitive agglutination. Accordingly, a new test is provided which can replace existing blood grouping and cross matching techniques.

The present invention measures agglutination using a process which is also capable of testing how tightly agglutinated cells are bonded by measuring how much force is required to separate them. This property depends upon antibodies interacting with the complement system. Agglutination of red blood cells is a function of the type and number of antigen combining sites on the surface of the cells, which bind with complementary Gig antibody molecules. The strength of agglutination is a function of the proximity of the binding sites on the cell surface. By placing a whole blood sample into a typically 1:10,000 suspension, and causing cells which are approximately bi-concave discs to sphere, the effective surface area available for bonding diminishes. Sphering a cell increases the space between antigen binding sites and increases the mean distance across which bonding occurs. The surface area available for bonding between cells decreases as cells sphere hence they lose bonding strength and separate. By recording the inducing pressure and the number of cells (or quantities related to it) as they change with respect to the inducing pressure, agglutination can be detected, quantified and monitored. Cells which have agglutinated, when tested by this method, separate and thereby increase the cell count in a characteristic fashion. In a further step the sample is subject to mechanical agitation which tends to promote agglutination in normally shaped cells capable of agglutination but promotes separation of spherically shaped cells.

### Brief Description of the Drawings

Examples of the present invention will now be described in detail with reference to the accompanying drawings, in which:
Figure 1 is a screen dump of a set of results from an automatic blood cell analyzer of the type described in detail in International patent application WO97/24601, for a patient having normal non-agglutinated blood cells;
Figure 2 is a similar screen dump of a set of results for a patient having agglutinated blood cells; and,
Figure 3 is another screen dump showing the results of mixing a sample of blood with antibodies in a test to determine blood type.

### Detailed Description

The method of the present invention is exceptionally useful in conjunction with the methods and apparatus described in the applicants' earlier filed International patent applications, namely WO97/24601, WO97/24598 and WO97/24599, and enhances the general utility of the tests described therein.

The preferred method consists of counting the cells as they pass through an aperture. The instrument may be configured with a mixing chamber into which saline, cells and diluent are injected, in which case the number of cells passing through the aperture at every osmolality does not vary. When only two streams are injected into the mixing chamber, diluent and a saline suspension into which the cells have been previously introduced, the number of cells passing through the aperture is fixed at a level that is directly proportional to the osmotic gradient. Since the red blood cells suspended in a liquid medium are exposed to a progressive reduction in ambient osmolality, and the method normally injects a progressively smaller stream of cells into the mixing chamber, a progressive reduction in cell count is observed.

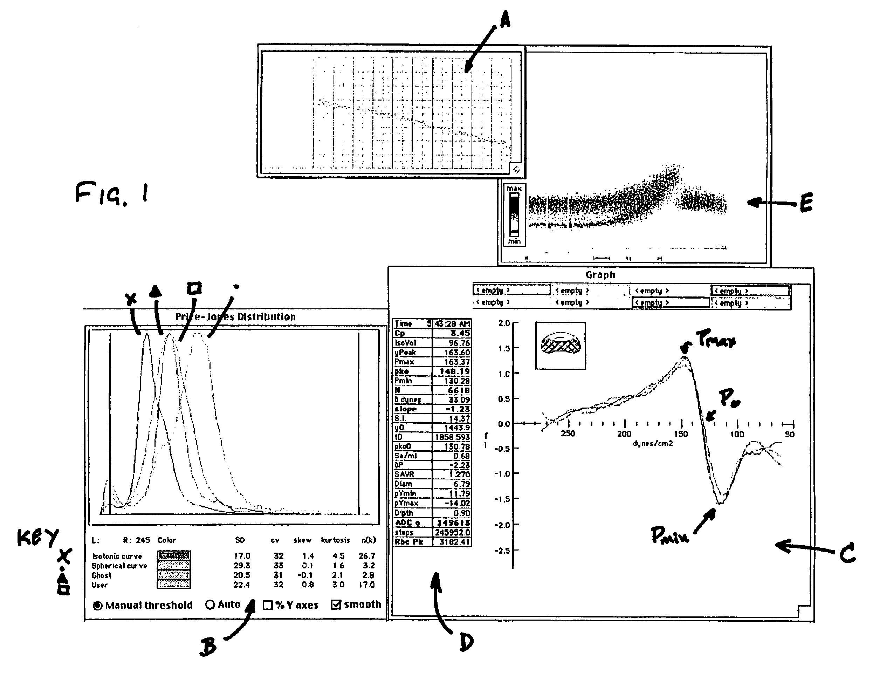

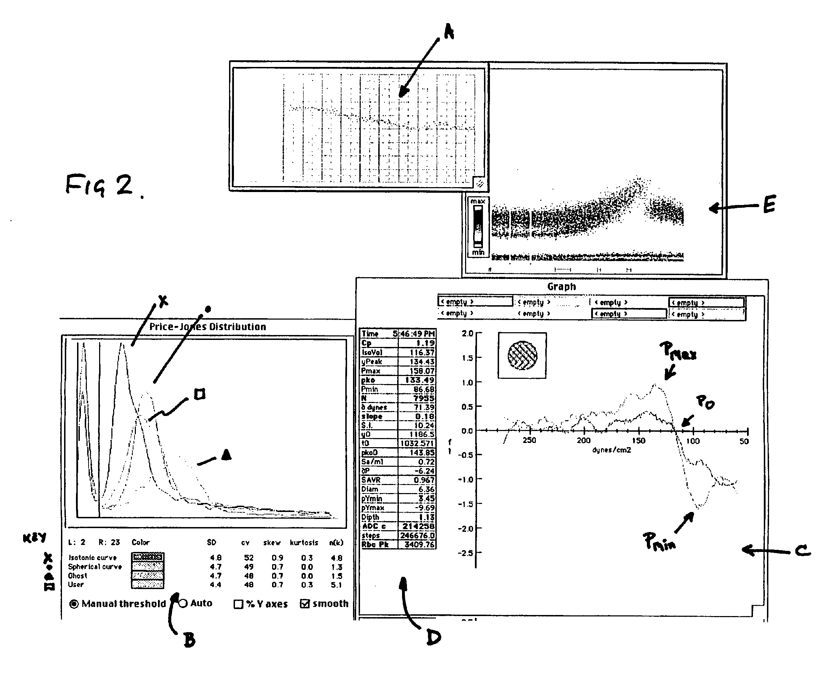

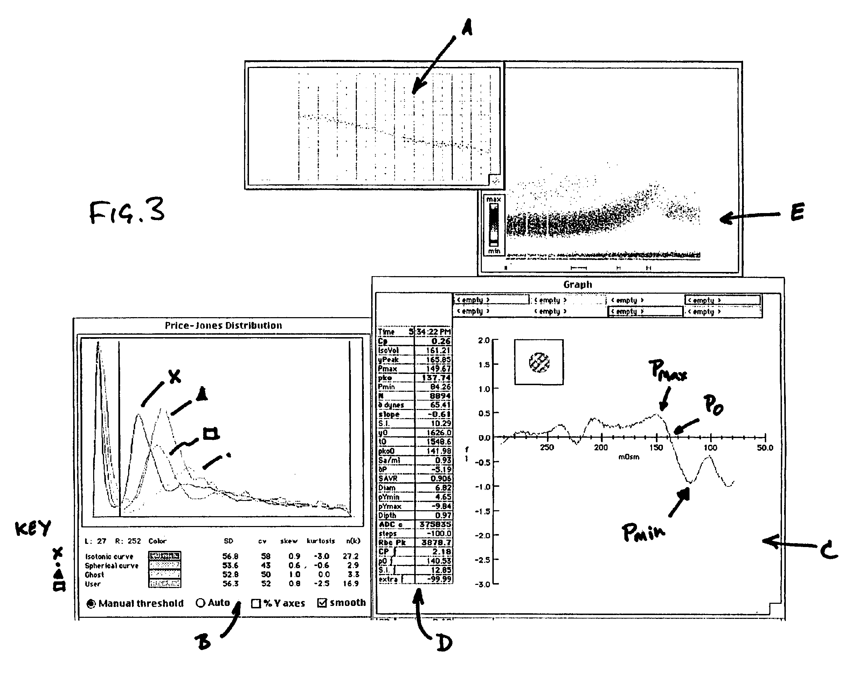

The results generated by the instrument described in International Patent Application WO 97/24601 for a normal patient are shown in Figure 1. In Figure 1, as well as in Figures 2 and 3, in area A the plot represents the red cell count. Deviation from the predetermined straight line of cell count against osmolality (as shown in area A of Figure 2) can only occur if additional particles appear, or are stimulated by the ambient change in pressure.

As will be described below, when cells agglutinate or are made to agglutinate, the cell count falls; then, as the cells sphere, the cell count increases with each aggregate tending to separate into its component parts in inverse proportion to the strength of the agglutination.

Most cells sphere in the range of pressures in the interval between Pₘₐₓ and P₀, where Pₘₐₓ is the point at which the rate of fluid flow into the cell reaches a maximum and P₀ is the equilibrium point (see area B). If agglutinated clumps are present they will separate in the same interval causing a local increase in count. Furthermore, the point at which P₀ occurs gives an indication of whether or not agglutination is occurring, since the point at which P₀ occurs increases if cells are agglutinating.

Our corresponding International application (Agent's reference G14201WO) discloses a method of measuring cell fragments. Fragments and disrupted agglutinated cells (DACs) can be segregated by size. Fragments are quite small between 10 and 30 fl in volume whereas DACs are at least three times the size, generally between 60-110 fl. In addition, the isotonic MCV is normal or reduced in the presence of fragments while the MCV is elevated with agglutination. As the normal range of MCV is so large it can hide much agglutination.

Sample ageing and the application of mechanical, ultrasound or other stress increases the count of intact cells if the sample was agglutinating and decreases the number of intact cells if the sample is fragmenting. Dropping the ambient osmolality below P₀ has no further disrupting effect on agglutinated clumps but the frequency of cell fragments have been found to vary inversely with osmolality.

Figure 2 shows the results for a patient having agglutinated blood cells. The sudden increase in cell count at sphering is shown clearly in area A, and the increased sphericity index (SI) appears as a fat cell in area B. SI can also be seen from the Table (area C). A sphere has a SI of 10 whereas a flatter cell has a higher SI. In Figure 2 (abnormal patient) the value of SI is 10.24 whereas in Figure 1 (healthy patient) the corresponding value is 14.37.

Area D in Figure 2, in comparison with Figure 1, shows the increase in variance of the red cell frequency distribution due to agglutinated clumps of cells. An analysis of the frequency distribution provides an indication of whether or not the cells are agglutinating. Firstly, the width of the distribution, as measured by the standard deviation (SD), or coefficient of variation (cv), increases with agglutination. Secondly, any deviation from a normal distribution can be measured. A bias away from the centre leading to a flatter shaped curve, termed negative kurtosis, provides an indication of agglutination. Comparing area D in Figures 1 and 2 shows that in the abnormal patient the standard deviations are about twice the normal and kurtosis is negative.

Area E shows frequency distributions indicating the profile of cell size measurement against increasing osmolality. As the stress is increased the cells begin to swell resulting in the gradual increase in mean cell size. At P₀ the cell size can increase no more, and upon a further increase in stress, the cells evacuate their contents to become "ghost cells".

As an example, the method may be embodied into an instrument for the automated recognition of blood groups and cell types by the induction and detection of agglutination by introducing antibodies (for example, using any one or more of the commercially available antibodies currently used for blood typing purposes, or using the recipient's plasma as the antibody source for the purpose of cross-matching) into the water syringe which subsequently meets the saline blood suspension in the mixing chamber. Agglutination caused by the interaction of the antibodies and the antigens on the surface of the cells, or the lack of it, is detected at the sensor aperture by counting. This test eliminates the need for manual blood grouping and cross-matching.

The results generated by such an instrument described in International patent application WO97/24601 are shown in Figure 3. In this example, the fluids were warmed within the apparatus to a temperature of around 37°C ie body temperature, to stimulate normal body environment. Agglutination is recognisable by the presence of an increase in the red cell count, usually between Pₘₐₓ and Pₘᵢₙ, by the frequency distribution (in this example the isotonic, spherical, ghost, and "user" selected frequency distributions are taken at respective sampling instants shown by the "H"s in area E) showing negative kurtosis, by an increase in the distribution width of the cell population measured by an increase in the standard deviation or coefficient of variation, by an increase in the sphericity index, and by an increase in the osmolality which induces zero permeability (P₀). Any change, even minor change (detectable when compared with a control run without antibodies) must be attributable solely to the antibody. In this particular example, a second population of cells is visible on both areas B and E in Figure 3, which also indicates agglutination.

The present invention is particularly useful in the early detection of agglutination, hence the early detection and subsequent treatment of haemolytic diseases, and enhanced possibility of recognizing the underlying pathology. It is also possible to quantify the strength of cell agglutination from the extent to which separation is achieved and the ease with which it is achieved. As the unagglutinated cell concentration is known any reduction in the isotonic count represents agglutination. As the cell suspension is exposed to the sphering gradient, the original count will be restored at higher osmolalities and in proportion to the strength of the agglutination. Finally, the method provides for the automatic identification of blood groups and cell types by inducing cells to agglutinate and subsequently testing them using the method.

## Claims

1. A method of detecting agglutination in a sample of cells, comprising the steps of inducing the cells to change at least one of their properties so as to separate agglutinated cells and detecting the resultant alteration in the cell population.

2. A method according to claim 1, comprising the step of measuring the force required to separate agglutinated cells.

3. A method according to claim 1 or 2, in which the property changed is that of the shape of the cells.

4. A method according to any preceding claim, in which the cell sample is subject to an alteration to cause the cells to sphere.

5. **A method according to claim 4, in which the alteration is a change in** osmolality of a liquid medium in which the cells are suspended.

6. A method according to any preceding claim, in which alterations in the cell population are detected by passing one or more aliquots of the cell sample through a sensor which is adapted to count the number of cells passing through the sensor.

7. A method according to claim 6, in which the sample is fed continuously into a solution the osmolality of which is changed continuously to produce a continuous series of aliquots of cells which are passed through the sensor.

8. A method according to any preceding claim, further comprising the step of pretreating the sample of cells to induce, or at least attempt to induce, agglutination.

9. A method according to any preceding claim, in which the cell sample is obtained from a source of whole blood.

10. A method according to claim 9, in which the sample of cells are treated with antibodies from a different source.

11. A method according to claim 10, in which the cells are treated in order to determine the blood type.

12. A method according to claim 10, in which the cells are treated in order to cross-match the sample.

13. A method according to any of claims 10 to 12, in which the antibodies from the different source are manufactured, or come from whole blood, plasma or serum.

14. A method according to any of claims 8 to 13, in which the sample of cells is pre-treated by exposure to heat.

15. A method according to any of claims 8 to 14, in which the sample is warmed to a temperature of between 35°C to 40°C, preferably 37°C.

16. A method according to any of claims 8 to 13, in which the sample of cells is pre-treated by cooling the sample.

## Patentansprüche

1. Verfahren zum Erfassen von Agglutination in einer Probe von Zellen, das die Schritte des Veranlassens einer Änderung wenigstens einer der Eigenschaften der Zellen, um **dadurch** agglutinierte Zellen abzutrennen, und des Erfassens der resultierenden Veränderungen der Zellenpopulation umfasst.

2. Verfahren nach Anspruch 1, das den Schritt des Messens der zum Abtrennen agglutinierter Zellen erforderlichen Kraft umfasst.

3. Verfahren nach Anspruch 1 oder 2, wobei die geänderte Eigenschaft die der Form der Zellen ist.

4. Verfahren nach einem der vorangehenden Ansprüche, wobei die Zellenprobe einer Veränderung ausgesetzt wird, um die Zellen zu veranlassen, Kugelform anzunehmen.

5. Verfahren nach Anspruch 4, wobei die Veränderung eine Änderung der Osmolalität eines flüssigen Mediums ist, in dem die Zellen suspendiert sind.

6. Verfahren nach einem der vorangehenden Ansprüche, wobei die Veränderungen in der Zellenpopulation erfasst werden, indem eine oder mehrere Aliquoten der Zellenprobe durch einen Sensor geleitet werden, der so eingerichtet ist, dass er die Zahl der durch den Sensor hindurchtretenden Zellen zählt.

7. Verfahren nach Anspruch 6, wobei die Probe kontinuierlich in eine Lösung geleitet wird, deren Osmolalität kontinuierlich geändert wird, um eine kontinuierliche Reihe von Aliqoten von Zellen zu erzeugen, die durch den Sensor hindurchgeleitet werden.

8. Verfahren nach einem der vorangehenden Ansprüche, das des weiteren den Schritt des Vorbehandelns der Probe von den Zellen umfasst, um Agglutination zu bewirken oder zumindest zu versuchen, sie zu bewirken.

9. Verfahren nach einem der vorangehenden Ansprüche, wobei die Zellenprobe von einer Quelle von Vollblut bezogen wird.

10. **Verfahren nach Anspruch 9, wobei die Probe von Zellen mit Antikörpern von einer** anderen Quelle behandelt wird.

11. Verfahren nach Anspruch 10, wobei die Zellen behandelt werden, um die Blutgruppe zu bestimmen.

12. Verfahren nach Anspruch 10, wobei die Zellen behandelt werden, um Einkreuzen der Probe durchzuführen.

13. Verfahren nach einem der Ansprüche 10 bis 12, wobei die Antikörper von der anderen Quelle aus Vollblut, Plasma oder Serum hergestellt werden oder davon stammen.

14. Verfahren nach einem der Ansprüche 8 bis 13, wobei die Probe von Zellen vorbehandelt wird, indem sie Wärme ausgesetzt wird.

15. Verfahren nach einem der Ansprüche 8 bis 14, wobei die Probe auf eine Temperatur zwischen 35 °C und 40 °C, vorzugsweise 37 °C, erwärmt wird.

16. Verfahren nach einem der Ansprüche 8 bis 13, wobei die Probe von Zellen vorbehandelt wird, indem die Probe gekühlt wird.

## Revendications

1. Procédé de détection d'une agglutination dans un échantillon de cellules, comprenant les étapes consistant à induire les cellules à modifier au moins une de leurs propriétés de façon à séparer des cellules agglutinées, et à détecter l'altération résultante de la population de cellules.

2. Procédé selon la revendication 1, comprenant l'étape consistant à mesurer la force requise pour séparer les cellules agglutinées.

3. Procédé selon la revendication 1 ou 2, dans lequel la propriété modifiée est la forme des cellules.

4. Procédé selon l'une quelconque des revendications précédentes, dans lequel l'échantillon de cellules est soumis à une altération pour amener les cellules à devenir sphérique.

5. Procédé selon la revendication 4, dans lequel l'altération consiste en une modification de l'osmolalité d'un milieu liquide dans lequel les cellules sont en suspension.

6. Procédé selon l'une quelconque des revendications précédentes, dans lequel des altérations de la population de cellules sont détectées en faisant passer une ou plusieurs aliquotes de l'échantillon de cellules à travers un détecteur adapté pour compter le nombre de cellules qui le traversent.

7. Procédé selon la revendication 6, dans lequel l'échantillon est amené en continu dans une solution dont l'osmolalité est modifiée en continu pour produire une série continue d'aliquotes de cellules qui sont envoyées à travers le détecteur.

8. Procédé selon l'une quelconque des revendications précédentes, comprenant en outre l'étape consistant à prétraiter l'échantillon de cellules pour induire, ou au moins tenter d'induire une agglutination.

9. Procédé selon l'une quelconque des revendications précédentes, dans lequel l'échantillon de cellules est obtenu à partir d'une source de sang complet.

10. Procédé selon la revendication 6, dans lequel l'échantillon de cellules est traité avec des anticorps provenant d'une source différente.

11. Procédé selon la revendication 10, dans lequel les cellules sont traitées pour déterminer le type sanguin.

12. Procédé selon la revendication 10, dans lequel les cellules sont traitées pour faire subir une épreuve de compatibilité à l'échantillon.

13. Procédé selon l'une quelconque des revendications 10 à 12, dans lequel les anticorps provenant de la source différente sont fabriqués, ou proviennent de sang complet, de plasma ou de sérum.

14. Procédé selon l'une quelconque des revendications 8 à 13, dans lequel l'échantillon de cellules est prétraité par exposition à la chaleur.

15. Procédé selon l'une quelconque des revendications 8 à 14, dans lequel l'échantillon est chauffé à une température entre 35°C et 40°C, de préférence 37°C.

16. Procédé selon l'une quelconque des revendications 8 à 13, dans lequel l'échantillon de cellules est prétraité par refroidissement de l'échantillon.
